# EUROPÄISCHE PATENTSCHRIFT

(11) **EP 3 394 022 B1**
(45) Veröffentlichungstag und Bekanntmachung des Hinweises auf die Patenterteilung: **18.09.2019**
(21) Anmeldenummer: 16822988.8
(22) Anmeldetag: 21.12.2016
(51) Int. Cl.: C07C 45/50, B01J 10/00, B01J 19/24

(54) **ZYLINDRISCHER REAKTOR UND DESSEN VERWENDUNG ZUR KONTINUIERLICHEN HYDROFORMYLIERUNG**
CYLINDRICAL REACTOR AND USE THEREOF FOR CONTINUOUS HYDROFORMYLATION
REACTEUR CYLINDRIQUE ET SON UTILISATION POUR L'HYDROFORMYLATION CONTINUE

(30) Priorität: 22.12.2015 EP 15202114
(43) Veröffentlichungstag der Anmeldung: 31.10.2018
(73) Patentinhaber: BASF SE, 67056 Ludwigshafen am Rhein (DE)
(72) Erfinder: PAPP, Rainer, 67346 Speyer (DE); GOBIN, Oliver Christian, 80689 Muenchen (DE); BEY, Oliver, 67150 Niederkirchen (DE); RUDOLPH, Jens, Abu Dhabi (AE); THELEN, Hans-Guenter, 68259 Mannheim (DE)
(74) Vertreter: BASF IP Association
(86) Internationale Anmeldenummer: PCT/EP2016/082057
(87) Internationale Veröffentlichungsnummer: WO 2017/108878

(56) Entgegenhaltungen:
- WO-A1-95/14647
- DE-A1- 19 836 807
- DE-A1- 19 854 637

## Beschreibung

### HINTERGRUND DER ERFINDUNG

Die vorliegende Erfindung betrifft einen zylindrischen Reaktor, der sich für exotherme, heterogene Reaktionen unter Einspeisung eines gasförmigen und eines flüssigen Eduktes und speziell für Hydroformylierungsreaktionen eignet. Die Erfindung betrifft weiterhin ein kontinuierliches Verfahren zur Herstellung von Aldehyden und/oder Alkoholen mit 6 bis 20 C-Atomen durch Hydroformylierung von Olefinen mit Synthesegas in Gegenwart eines im flüssigen Reaktionsmedium homogen gelösten Übergangsmetallkatalysators in einem solchen Reaktor.

### STAND DER TECHNIK

Die Hydroformylierung oder Oxo-Synthese ist ein wichtiges großtechnisches Verfahren und dient der Herstellung von Aldehyden aus Olefinen und Synthesegas, d. h. einem Gemisch aus Kohlenmonoxid und Wasserstoff. Die erhaltenen Aldehyde können gegebenenfalls im gleichen Arbeitsgang oder nachfolgend in einem getrennten Hydrierschritt mit Wasserstoff zu den entsprechenden Alkoholen hydriert werden. Das Verfahren findet in der Regel unter homogener Katalyse statt, wobei ein Übergangsmetallkatalysator eingesetzt wird, der in der flüssigen Phase des Reaktionsgemischs gelöst ist. Dem Fachmann steht dafür ein breites Angebot bewährter Oxosyntheseverfahren zur Verfügung (vgl. J. Falbe, Herausgeber, "New Syntheses with Carbon Monoxide", Springer-Verlag, New York 1980, Seite 162 - 174). Während niedere Olefine inzwischen fast ausschließlich mit ligandenmodifizierten Rhodium- bzw. Cobaltkatalysatoren, beispielsweise mit phosphinmodifizierten Rhodiumkatalysatoren, hydroformyliert werden, behauptet die Oxosynthese mit unmodifizierten Cobalt- oder Rhodiumkatalysatoren bei der Umsetzung der höheren Olefine (d. h. von Olefinen mit mehr als 6 C-Atomen) nach wie vor ihre dominierende Stellung. Unter unmodifizierten Katalysatoren werden solche verstanden, die außer CO und H₂ keine weiteren Liganden, speziell Phosphororganylverbindungen (wie z. B. Triphenylphosphin), aufweisen.

Mit Ausnahme der unverzweigten Olefine, bei denen der Erhalt der linearen Struktur bei der Hydroformylierung von gewichtiger Bedeutung für die Endprodukte ist, werden die durch Oligomerisierung von C₃- und/oder C₄-Olefinen gut zugänglichen verzweigten Olefine großtechnisch fast ausschließlich mit unmodifizierten Cobaltcarbonylen, d. h. ohne Gegenwart von zusätzlichen, organischen, phosphorhaltigen Liganden, hydroformyliert. Dieses Katalysatorsystem ist nicht nur sehr preiswert, sondern auch universell einsetzbar. Außerdem erhält man mit unmodifizierten Cobaltkatalysatoren im Vergleich zu unmodifizierten Rhodiumkatalysatoren ausgehend von dem gleichen längerkettigen Ausgangsolefin höhere Ausbeuten an den besonders begehrten linearen Aldehyden.

Die Umsetzung von Olefinen mit Kohlenmonoxid und Wasserstoff zu Aldehyden und Alkoholen verläuft exotherm. Bei einer kontinuierlichen Durchführung dieser Reaktion im großtechnischen Maßstab muss man daher Vorkehrungen treffen, um die Reaktionswärme im Reaktionsgemisch gleichmäßig zu verteilen und abzuführen. Lokale Temperaturspitzen, die zu unerwünschten Neben- und Folgereaktionen führen, müssen dabei weitgehend vermieden werden. Außerdem muss gewährleistet werden, dass die einerseits flüssigen und andererseits gasförmigen Ausgangsstoffe gut durchmischt werden, damit die Reaktion mit möglichst hohem Umsatz abläuft.

Die gegenwärtig großtechnisch eingesetzten Verfahren unterscheiden sich im Wesentlichen darin, in welcher Form der Cobaltkatalysator dem Hydroformylierungsreaktor zur Verfügung gestellt wird und auf welche Weise der Katalysator nach erfolgter Hydroformylierung aus dem Reaktionsgemisch entfernt und möglichst verlustfrei wieder in den Prozess zurückgeführt wird.

Eine bewährte Methode ist dabei der Einsatz wässriger Lösungen von Cobaltsalzen niederer Carbonsäuren, vorzugsweise Cobaltformiat oder Cobaltacetat. Unter den im Reaktor herrschenden Bedingungen werden die Co(II)-Salze rasch in den eigentlichen Hydroformylierungskatalysator, Cobaltcarbonylwasserstoff (HCo(CO)₄), überführt. Die Hydroformylierung erfolgt in einem ein inniges Gemisch aus Olefin, Katalysatorkomplex und Synthesegas enthaltenden Hochdruckreaktor bei erhöhten Temperaturen, beispielsweise von 120 bis 210 °C und erhöhten Drücken, beispielsweise von 100 bis 400 bar. Nach erfolgter Umsetzung des Olefins können die zuvor in der organischen Phase homogen gelösten Cobaltcarbonyle durch einen Wertigkeitswechsel am Zentralatom mit Hilfe von oxidierenden Substanzen wieder in Co(II)-Verbindungen überführt werden. Mit einer schwach sauren, wässrigen Phase werden die Co(II)-Verbindungen aus dem organischen Reaktionsprodukt extrahiert, so dass dieses nach Phasenscheidung praktisch Cobalt-frei ist und direkt der Weiterverarbeitung zugeführt werden kann.

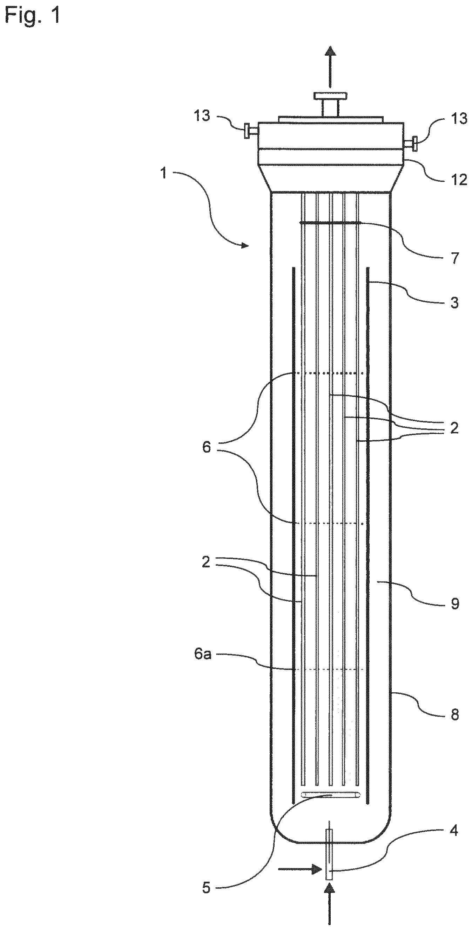

Problematisch ist, dass der als Katalysator fungierende Cobaltcarbonylwasserstoff in Abhängigkeit von der Cobaltkonzentration und der Temperatur nur oberhalb eines jeweils bestimmten Kohlenmonoxidpartialdruckes stabil ist, unterhalb desselben jedoch als metallisches Cobalt ausfällt, das sich im Reaktor niederschlägt, zu Katalysatorverlusten und zu Verstopfungen, insbesondere in Rohrleitungen und Armaturen führt. Ein Stabilitätsdiagramm des Cobaltcarbonylwasserstoffes in Abhängigkeit von den Variablen Temperatur und Kohlenmonoxidpartialdruck ist in der Monographie von J. Falbe (Herausgeber): "New Synthesis with Carbon Monoxide", Springer Verlag, Berlin, 1980, S. 17, Fig. 1.9, wiedergegeben.

Großtechnische Hydroformylierungsverfahren müssen somit nicht nur eine möglichst gleichförmige Durchmischung der Edukte, d. h. der organischen flüssigen Olefinphase, der gasförmigen Phase, umfassend Kohlenmonoxid und Wasserstoff, und gegebenenfalls der wässrigen, Cobalt enthaltenden Katalysatorlösung sowie die gleichförmige Verteilung und schnelle Abführung der Reaktionswärme gewährleisten, sondern darüber hinaus auch sicherstellen, dass der Cobaltkatalysator in der aktiven Form als Cobaltcarbonylwasserstoff im Wesentlichen über die Reaktionszone homogen verteilt vorliegt.

Ein in der Technik häufig genutztes Verfahren zur Abführung der Reaktionswärme über einen außenliegenden Wärmetauscher kann die obigen Voraussetzungen nicht erfüllen, da aktiver Katalysator im externen Kreislauf auf Grund des dort vorliegenden niedrigeren Kohlenmonoxidpartialdruckes ausfallen würde.

Es wurde daher eine Vielzahl von Verfahren entwickelt, die eine Abführung der Reaktionswärme innerhalb des Hydroformylierungsreaktors gewährleisten.

In DE 1 135 879 wird beschrieben, dass im Inneren des Reaktors ein frei stehendes Umlaufrohr enthalten sein kann, das eine zirkulierende Konvektionsströmung verursacht. Weiterhin kann der Reaktor im Inneren mit Kühlrohren versehen sein, in denen die Reaktionswärme zur Dampferzeugung nutzbar gemacht werden kann. Die Reaktions- und Verfahrensparameter werden detailliert beschrieben, nicht aber der weitere Aufbau des Reaktors.

Dokument DE 1 205 514 beschreibt, dass man zumindest die flüssigen Reaktionsteilnehmer mit hoher Geschwindigkeit in den Reaktor führt, insbesondere durch Einspritzdüsen.

In DE 1 938 102 sind Umlaufapparaturen mit senkrecht stehendem Hochdruckreaktor mit einer Mischzone oder Bündeln von Mischzonen von unterschiedlichen Durchmessern und die Einführung von Reaktionsteilnehmern mit einer Düse oder einem Bündel von Düsen in die Reaktionszone, bekannt. Die Mischzone kann insbesondere als zylindrisches Rohr oder Kegelsegment und die Düse als Schlitz- oder Ringdüse ausgestaltet sein. Auch wird beschrieben, dass organische Stoffe oder Wasser als Kühlflüssigkeit eingesetzt werden können. Dabei wird der Siedepunkt durch den eingestellten Druck beeinflusst. Die Kühlzone wird mit so viel Flüssigkeit beschickt, dass die Wärmeübertragerflächen immer benetzt sind. Es wird bevorzugt eine Temperaturdifferenz von 20 bis 100 °C zwischen dem Kühlmittel und dem Reaktorinhalt eingesetzt. Nicht beschrieben ist die Anordnung von Fieldrohren und/oder weiteren Vorrichtungen innerhalb des senkrecht stehenden Hochdruckreaktors.

In DE 1 938 104 wird ein senkrecht stehender Hochdruckreaktor mit einem Umlaufrohr und einer Eindüsung durch eine Mehrstoffdüse beschrieben. Der Oxoreaktor weist ein Kühlsystem mit einer mittleren Fläche von 190 m² auf, wobei das Kühlsystem mit Methanol betrieben wird. Nicht bekannt ist die Anordnung von Fieldrohren und/oder weiteren Vorrichtungen innerhalb des senkrecht stehenden Hochdruckreaktors.

In DE 198 54 637 wird ein Reaktor in einer hochzylindrischen Bauform mit einer im oberen Reaktorbereich angeordneten nach unten gerichteten Strahldüse beschrieben, wobei innerhalb des Reaktors ein Leitrohr über die gesamte Länge konzentrisch angeordnet ist. In einer Ausführungsform ist auf der der Strahldüse abgewandten Seite des Leitrohres eine Prallplatte angeordnet. Außerhalb des Leitrohres können im Ringraum Wärmetauscher, insbesondere Fieldrohr-Wärmetauscher angeordnet sein. Es wird ein Verfahren zur kontinuierlichen Durchführung von Gas-Flüssig- und Flüssig-Flüssig-Reaktionen beschrieben. Die Zufuhrströmungsrichtung in den Reaktor erfolgt in Gravitationsrichtung und es ist keine Anordnung von Fieldrohren innerhalb des Leitrohres des Reaktors beschrieben.

In DE 101 26 363 wird ein Hydroformylierungsreaktor mit einer Düse mit einem verstellbaren Düsendurchlassquerschnitt beschrieben, wobei keine Einrichtungen zur Wärmeabfuhr dargestellt sind. Durch die verstellbare Düse kann der Leistungseintrag verändert werden. Dies erlaubt es unter anderem, diesen an die in einer Anlage gefahrene Last anzupassen. In dem senkrecht stehenden Hochdruckrohr können ein oder mehrere Einbauten angeordnet sein, welche eine oder mehrere Mischzonen definieren. Ein Leitrohr und oder eine Anordnung von Fieldrohren innerhalb des Leitrohres des Reaktors ist nicht beschrieben.

Dokument EP 2 043 774 beschreibt einen Hydroformylierungsreaktor mit innenliegender Kühlung mittels Fieldrohren, die in Reaktorlängsrichtung kreisförmig angeordnet und mittels Blechen fest miteinander verbunden sind, dergestalt, dass sie ein Umlaufrohr ausbilden. Diese Konstruktion weist insbesondere die folgenden Nachteile auf:
Aufwendige Fertigung und Reparatur sowie geringe Flexibilität, da die Zahl der Rohre und damit die Wärmeübertragungsfläche begrenzt sind und die Breite des äußeren Ringraumes zwischen Umlaufrohr und Reaktorinnenmantel nicht ohne weiteres eingestellt werden kann.

Bekannte Rohrreaktoren zur Hydroformylierung von Olefinen haben insbesondere die Nachteile einer begrenzten Wärmeübertragungsfläche, einer geringen Variabilität der Einstellung der Wärmeübertragungsfläche, von niedrigen Durchströmungsgeschwindigkeiten, Rückströmungen in Leitrohren der Reaktoren und daraus resultierend Ausfällungen von Katalysatoren und den Nachteil einer aufwendigen Fertigung und Reparatur.

Es war daher Aufgabe der vorliegenden Erfindung, einen verbesserten Reaktor mit zentralem Einsteckrohr zur Hydroformylierung von Olefinen mit einer stabilen Betriebsweise bereitzustellen, der die zuvor genannten Nachteile nicht aufweist. Insbesondere soll eine fluide Rückströmung innerhalb des Einsteckrohres und eine Ausfällung des Katalysators vermieden werden. Des Weiteren soll eine ausreichende Wärmeübertragungsfläche im Rohrreaktor bereitgestellt werden und eine hohe Durchmischung der Reaktionsedukte gewährleistet sein.

Die Aufgabe wird gelöst durch einen zylindrischen Reaktor mit vertikaler Längsachse
- mit einer Vielzahl von Fieldrohren, die parallel zur Längsachse des Reaktor ausgerichtet und am oberen Ende des Reaktors befestigt sind,
- mit einem an beiden Enden offenen Einsteckrohr, das die Fieldrohre umhüllt und an seinem unteren Ende über dieselben hinausragt,
- mit einer Einlassdüse oder mehreren Einlassdüsen am Boden des Reaktors zum Eindüsen eines Eduktgemisches, das wenigstens eine gasförmige und wenigstens eine flüssige Komponente enthält, wobei
- die Fieldrohre in ihrer Anzahl und ihren Abmessungen dergestalt ausgelegt sind, dass
   - die Gesamtwärmeübertragungsfläche derselben bezogen auf das Innenvolumen des Reaktors im Bereich von 1 m²/m³ bis 12 m²/m³ und
   - die von den Fieldrohren belegte Querschnittsfläche bezogen auf die Querschnittsfläche des Einsteckrohres im Bereich von 0,03 m²/m² bis 0,30 m²/m² liegt.

Ein weiterer Gegenstand der Erfindung ist ein kontinuierliches Verfahren zur Herstellung von C₇-C₂₁-Oxoprodukten durch Hydroformylierung wenigstens eines C₆-C₂₀-Olefins mit Synthesegas in Gegenwart eines homogen gelösten Übergangsmetallkatalysators, wobei das Verfahren in einem zylindrischen Reaktor mit vertikaler Längsachse durchgeführt wird,
- mit einer Vielzahl von Fieldrohren, die parallel zur Längsachse des Reaktor ausgerichtet und in einem Rohrboden am oberen Ende des Reaktors eingeschweißt sind,
- mit einem an beiden Enden offenen Einsteckrohr, das die Fieldrohre umhüllt und an seinem unteren Ende über dieselben hinausragt,
- mit einer Einlassdüse oder mehreren Einlassdüsen am Boden des Reaktors zum Eindüsen eines Eduktgemisches, enthaltend das C₆-C₂₀-Olefin, das Synthesegas und gegebenenfalls den Übergangskatalysator, wobei
- die Fieldrohre in ihrer Anzahl und ihren Abmessungen dergestalt ausgelegt sind, dass
   - die Gesamtwärmeübertragungsfläche derselben bezogen auf das Innenvolumen des Reaktors im Bereich von 1 m²/m³ bis 12 m²/m³ liegt und
   - die von den Fieldrohren belegte Querschnittsfläche bezogen auf die Querschnittsfläche des Einsteckrohres im Bereich von 0,03 m²/m² bis 0,30 m²/m² liegt.

Die folgenden Ausführungen zu geeigneten und bevorzugten Ausgestaltungen des erfindungsgemäßen zylindrischen Reaktors gelten gleichermaßen für den in dem erfindungsgemäßen Verfahren zur Herstellung von C₇-C₂₁-Oxoprodukten eingesetzten Reaktor.

Der Begriff C₇-C₂₁-Oxoprodukte bezeichnet die bei der Hydroformylierung (Oxo-Reaktion) von C₆-C₂₀-Olefinen erhaltenen C₇-C₂₁-Aldehyde und C₇-C₂₁-Alkohole.

Im Rahmen der vorliegenden Erfindung wird unter dem Innenvolumen des Reaktors das gesamte Reaktorvolumen einschließlich des Volumens der im Reaktor befindlichen Einbauten verstanden. Das Innenvolumen ist somit größer als das Reaktionsvolumen, d. h. das Volumen des Reaktors, das theoretisch mit einem Reaktionsmedium gefüllt werden kann.

Der erfindungsgemäße Reaktor kombiniert das bekannte Prinzip eines Schlaufenreaktors mit interner Umwälzung des Reaktionsgemisches, die durch Zuführung des Eduktgemisches über eine oder mehrere Einlassdüse(n) in den von einem konzentrisch zum Reaktorinnenmantel angeordneten Einsteckrohr (auch häufig als Leit- oder Umlaufrohr bezeichnet) abgegrenzten Innenraum angetrieben wird mit einer Kühlung durch im vom Einsteckrohr abgegrenzten Innenraum angeordneten Fieldrohren.

Umlaufreaktoren sind bekannt und beispielsweise in der DE 198 54 637 beschrieben. Umlaufreaktoren sind Reaktoren in hochzylindrischer Bauform, das heißt mit vertikaler Längsachse, und weisen als wesentliche Einbauelemente ein konzentrisch zum Reaktorinnenmantel angeordnetes und sich im Wesentlichen über die gesamte Reaktorlänge, bis auf die Reaktorenden, erstreckendes Einsteckrohr auf sowie eine oder mehrere Strahldüsen, die das Eduktgemisch in den vom Einsteckrohr abgegrenzten Reaktorinnenraum eindüsen. Dadurch wird eine gerichtete interne Umlaufströmung ausgebildet, mit den Vorteilen, dass im gesamten Reaktorvolumen die Strömungsbedingungen eindeutig definiert sind, das heißt, dass die für die Auslegung des Reaktors wichtigen Größen Strömungsgeschwindigkeiten, Gasgehalte, Rückvermischung, Mischzeiten und Verweilzeitverhalten ausreichend präzise erfasst werden können und der Reaktor entsprechend unmittelbar scale-up-fähig ist.

Bei dem erfindungsgemäßen Reaktor wird die interne Umlaufströmung durch Eindüsen des Reaktionsgemisches über eine am unteren Ende des Reaktors angeordnete Strahldüse angetrieben, die eine vertikal von unten nach oben gerichtete Durchströmung des durch das Einsteckrohr abgegrenzten Reaktorinnenraumes bewirkt.

In den zylindrischen Reaktor werden über die Einlassdüse oder über mehrere Einlassdüsen ein gasförmiges und ein flüssiges Edukt eingespeist. Teilströme des gasförmigen und/oder des flüssigen Eduktes können dem Reaktor gewünschtenfalls zusätzlich über wenigstens eine weitere von den Einlassdüsen verschiedene Einspeisevorrichtung zugeführt werden. Das Edukt kann dabei z. B. in Form eines zweiphasigen Gemischs, das eine flüssige und eine gasförmige Phase aufweist, oder in Form eines dreiphasigen Gemischs, das eine gasförmige und zwei nicht (vollständig) mischbare flüssige Phasen aufweist, vorliegen.

Wird der Reaktor zur Hydroformylierung eingesetzt, so wird über die Einlassdüse oder mehrere Einlassdüsen in den Reaktor ein zwei- oder dreiphasiges Eduktgemisch, umfassend C₆-C₂₀-Olefine als flüssige organische Phase, eine gasförmige Mischung von Kohlenmonoxid und Wasserstoff (Synthesegas) und gegebenenfalls eine wässrige Lösung, die den Übergangsmetallkatalysator enthält, eingedüst. Wie im Folgenden ausgeführt, kann der Katalysator auch präformiert und mit der flüssigen organischen Phase in den Reaktor eingespeist werden.

Bevorzugt weist der zylindrische Reaktor wenigstens eine Einlassdüse zum Einspeisen des Eduktgemisches auf, die als Strahldüse ausgebildet ist.

Der Begriff Strahldüse bezeichnet in bekannter Weise ein sich in Strömungsrichtung verjüngendes Rohr. In der Regel wird eine einzige Strahldüse, die zentral am Boden des Reaktors angeordnet ist, eingesetzt. Es ist jedoch auch möglich, anstelle einer einzelnen Strahldüse mehrere, insbesondere regelmäßig um die Reaktorachse angeordnete Strahldüsen, einzusetzen. Vorteilhaft wird eine Ejektorstrahldüse eingesetzt, das heißt eine Strahldüse, die die kinetische Energie eines Flüssigkeitsstrahles hoher Geschwindigkeit zum Ansaugen und Dispergieren einer oder mehrerer Mischungskomponente(n) nutzt. Die Mischungskomponenten sind z. B. ausgewählt unter C₆-C₂₀-Olefinen, Synthesegas und wässrigen Übergangsmetallkatalysatorlösungen. Durch den hohen Energieeintrag werden in einer Ejektorstrahldüse hohe Turbulenzen und große Scherkräfte erzeugt, wodurch eine sehr gute Durchmischung erreicht wird.

Vorteilhaft kann als Strahldüse eine Düse mit einstellbarem Durchlassquerschnitt eingesetzt werden, wie sie in DE 101 26 363 beschrieben ist, und wodurch gewährleistet werden kann, dass hohe Eintrittsgeschwindigkeiten von 10 bis 80 m/s, insbesondere von 50 bis 70 m/s für das Eduktgemisch in den Reaktor auch unter wechselnden Belastungen ohne zusätzliche externe Kreislaufpumpen aufrecht erhalten werden können.

Als Substrate für das erfindungsgemäße Hydroformylierungsverfahren kommen prinzipiell alle Verbindungen in Betracht, welche eine oder mehrere ethylenisch ungesättigte Doppelbindung(en) enthalten. Das erfindungsgemäße Verfahren eignet sich vorteilhaft zur Hydroformylierung von Olefinen oder Olefingemischen mit wenigsten 6 Kohlenstoffatomen pro Molekül. Bevorzugt wird ein C₆-C₂₀-Olefin oder eine Mischung von C₆-C₂₀-Olefinen eingesetzt. In einer speziellen Ausführung wird ein C₈-C₁₆-Olefin oder eine Mischung von C₈-C₁₆-Olefinen eingesetzt.

Dazu zählen z. B. Olefine, wie α-Olefine, interne geradkettige und interne verzweigte Olefine. Geeignete α-Olefine sind z. B. 1-Hexen, 1-Hepten, 1-Octen, 1-Nonen, 1-Decen, 1-Undecen, 1-Dodecen, etc.

Geeignete geradkettige Olefine mit internen Doppelbindungen sind z. B. 2-Hexen, 3-Hexen, 2-Hepten, 3-Hepten, 2-Octen, 3-Octen, 4-Octen, etc.

Geeignete verzweigte Olefine mit internen Doppelbindungen sind z. B. 2-Methyl-2-penten, 3-Methyl-2-penten, verzweigte Hepten-Gemische mit internen Doppelbindungen, verzweigte Octen-Gemische mit internen Doppelbindungen, verzweigte Nonen-Gemische mit internen Doppelbindungen, verzweigte Decen-Gemische mit internen Doppelbindungen, verzweigte Undecen-Gemische mit internen Doppelbindungen, verzweigte Dodecen-Gemische mit internen Doppelbindungen, etc.

Das erfindungsgemäße Verfahren ist insbesondere für die Hydroformylierung von isomeren Olefingemischen geeignet, die durch Oligomerisierung von niederen Olefinen, wie Propen und Butenen, hergestellt werden. Zu den typischen Oligomeren, die als Ausgangsprodukte für das vorliegende Verfahren geeignet sind, zählen unter anderem Di-, Tri- und Tetrapropen, Di-, Tri- und Tetrabuten sowie Mischoligomere von Propenen und Butenen. Die Oligomere von Butenen sind nach bekannten Oligomerisierungsverfahren, z. B. nach dem Octol ®-Prozess von Hüls und dem Dimersol TM-Verfahren von IFP, großtechnisch zugänglich. Nach dem erfindungsgemäßen Verfahren können ferner lineare langkettige Olefine mit endständiger Doppelbindung, die z. B. nach dem SHOP®-Prozess oder Ziegler-Verfahren erhältlich sind, oder lineare langkettige Olefine mit innenliegender Doppelbindung hydroformyliert werden. Geeignete Ausgangsprodukte für das vorliegende Verfahren sind auch die gering verzweigten Oligomere, wie sie nach dem in der WO 95/14647 beschriebenen Verfahren erhalten werden. Danach werden unverzweigte C₂-C₆-Olefine einer Oligomerisierung an einem Katalysator unterzogen, der als aktive Bestandteile 10 bis 70 Gew.-% Nickeloxid, berechnet als NiO, 5 bis 30 Gew.-% Titandioxid und/oder Zirkoniumdioxid, 0 bis 20 Gew.-% Aluminiumoxid, 20 bis 40 Gew.-% Siliciumdioxid und 0,01 bis 1 Gew.-% eines Alkalimetalloxids enthält.

Als weiteres Edukt für das erfindungsgemäße Verfahren wird sogenanntes Synthesegas, das heißt eine Mischung aus Kohlenmonoxid und Wasserstoff, eingesetzt. Die Zusammensetzung desselben kann in weiten Bereichen variieren.

Bevorzugt beträgt das molare Verhältnis von Kohlenmonoxid und Wasserstoff etwa 10 : 1 bis 1 : 10, insbesondere 2,5 : 1 bis 1 : 2,5. Ein bevorzugtes Verhältnis liegt bei etwa 4 : 6.

Das erfindungsgemäße Verfahren findet unter homogener Katalyse statt. In der Regel wird hierzu mit dem Olefin und dem Synthesegas ein geeigneter Katalysator oder Katalysatorvorläufer in den Reaktor eingeführt. Bevorzugt umfassen die Katalysatoren oder Katalysatorvorläufer ein Metall der Gruppe VIII B des Periodensystems der Elemente, besonders bevorzugt Cobalt oder Rhodium, insbesondere Cobalt. Bevorzugt wird ein homogen im Reaktionsmedium gelöster Metall-Carbonylkomplexkatalysator, speziell ein Cobalt-Carbonylkomplexkatalysator, eingesetzt. Sofern nicht bereits die katalytisch aktive Spezies dem Reaktor zugeführt wird, werden im Allgemeinen unter den Hydroformylierungsbedingungen aus den jeweils eingesetzten Katalysatorvorstufen katalytisch aktive Spezies gebildet, wobei es sich in der Regel um Übergangsmetallcarbonyl- oder Übergangsmetallcarbonylwasserstoff-Verbindungen handelt.

Geeignete Cobalt-Katalysatorvorläufer sind beispielsweise Cobalt(II)chlorid, Cobalt(II)-sulfat, Cobalt(II)carbonat, Cobalt(II)nitrat, deren Amin- oder Hydratkomplexe oder Cobaltcarboxylate, z. B. Cobalt(II)-formiat, Cobalt(II)-acetat oder Cobalt(II)-ethylhexanoat. Geeignet sind weiterhin Cobaltkomplexe, insbesondere Cobaltcarbonyle oder Cobaltcarbonylwasserstoffe.

Geeignete Rhodium-Katalysatorvorläufer oder -komplexe sind z. B. Rhodium(II)- und Rhodium(III)-salze, wie Rhodium(III)-chlorid, Rhodium(III)-nitrat, Rhodium(III)-sulfat, Kalium-Rhodiumsulfat, Rhodium(II)- bzw. Rhodium(III)-carboxylat, Rhodium(II)- und Rhodium(III)-acetat, Rhodium(III)-oxid, Salze der Rhodium(III)-säure, Trisammoniumhexachlororhodat(III) etc. Weiterhin eignen sich Rhodiumkomplexe, wie Rhodiumbiscarbonylacetylacetonat, Acetylacetonatobisethylenrhodium(I) etc. Vorzugsweise werden Rhodiumbiscarbonylacetylacetonat oder Rhodiumacetat als Katalysatorvorläuferverbindung eingesetzt.

Der Katalysator oder seine Vorläuferverbindung kann dem Reaktor in gelöster Form im Einsatzolefin, einem organischen Lösungsmittel oder in Wasser oder einer Mischung davon, zugeführt werden. In einer speziellen Ausführung wird der Katalysator vor seiner Einspeisung in den Reaktor präformiert. Dazu kann man z. B. eine wässrige Cobalt(II)-salzlösung mit Synthesegas in Kontakt bringen, wobei ein hydroformylierungsaktiver Cobaltkatalysator gebildet wird. Die den Cobaltkatalysator enthaltende wässrige Lösung kann gleichzeitig oder anschließend mit dem Einsatzolefin und/oder dem externen organischen Lösungsmittel in Kontakt gebracht werden, wobei der Cobaltkatalysator in die organische Phase extrahiert wird. Diese wird dann dem Reaktor zugeführt.

Die Reaktion erfolgt in flüssiger Phase, wobei das Reaktionsgemisch nicht umgesetzte Olefine, gelöstes Synthesegas, den homogen gelösten Hydroformylierungskatalysator, mindestens einen Aldehyd als Hydroformylierungsprodukt sowie gegebenenfalls Nebenprodukte der Hydroformylierungsreaktion enthält. Nebenprodukte bei der Oxoreaktion sind z. B. die Produkte der Aldolreaktion der gebildeten Aldehyde. Geeignete externe organische Lösungsmittel sind inerte Kohlenwasserstoffe, wie Paraffinfraktionen, aromatische Kohlenwasserstoffe, wie Benzol, Toluol oder Xylol. Bevorzugt dienen jedoch die bei der Hydroformylierung gebildeten Aldehyde und/oder Alkohole und die hochsiedenden Nebenprodukte der Hydroformylierung als Lösungsmittel.

Die Temperatur bei der Hydroformylierung liegt im Allgemeinen bei 100 bis 250 °C, insbesondere 145 bis 200 °C. Die Hydroformylierung wird vorzugsweise bei einem Druck im Bereich von 10 bis 400 bar, besonders bevorzugt 20 bis 350 bar, insbesondere 100 bis 300 bar, durchgeführt.

Die Reaktionswärme wird durch indirekten Wärmetausch innerhalb des Reaktors über innerhalb des vom Einsteckrohr umgrenzten Reaktorinnenraumes angeordnete Fieldrohre abgeführt. Als Fieldrohre (oder Siederohre) werden im Rahmen der Erfindung Doppelmantelrohre mit einem inneren, beidseitig offenen Rohr bezeichnet, über das die Kühlflüssigkeit, von oben nach unten geführt wird, das im Zwischenraum zwischen dem inneren, unten offenen, und dem äußeren, unten geschlossenen Rohr, teilweise verdampft und als Dampf-/Wassergemisch am oberen Ende desselben abgezogen wird. Dabei wird dem Reaktionsgemisch die durch die exotherme Reaktion entstehende Wärme entzogen. Als Kühlmittel können dafür übliche Flüssigkeiten oder Gase eingesetzt werden. Bevorzugt wird als Kühlmittel Wasser eingesetzt, beispielsweise enthärtetes und entgastes Wasser (sogenanntes Kesselspeisewasser).

Erfindungsgemäß sind die Fieldrohre dergestalt ausgelegt, dass sie einerseits eine ausreichende Gesamtwärmeübertragungsfläche aufweisen, so dass die Wärme der stark exothermen Reaktion ausreichend schnell abgezogen wird, dass sie jedoch gleichzeitig die Querschnittsfläche des Einsteckrohrs nur insoweit belegen, dass die interne Umlaufströmung nicht zu stark behindert wird und dass es nicht zu einer fluiddynamischen Einschnürung (Käfigeffekt) im Einsteckrohr kommt.

Als Gesamtwärmeübertragungsfläche wird vorliegend die Summe der Wärmeübertragungsflächen sämtlicher Fieldrohre verstanden, soweit sie in Kontakt mit dem Reaktionsgemisch sind. Erfindungsgemäß liegt die Gesamtwärmeübertragungsfläche der Fieldrohre bezogen auf das Innenvolumen des Reaktors im Bereich von 1 m²/m³ bis 12 m²/m³. Bevorzugt liegt die Gesamtwärmeübertragungsfläche der Fieldrohre bezogen auf das Innenvolumen des Reaktors im Bereich von 7 bis 11 m²/m³. Erfindungsgemäß sind die Fieldrohre so ausgestaltet, dass sie den im Reaktor herrschenden Reaktionsbedingungen standhalten. Wird der Reaktor zur Hydroformylierung eingesetzt, so sind die Fieldrohre so ausgelegt, dass sie den im Reaktor herrschenden Drücken bis ca. 400 bar und Temperaturen bis ca. 250 °C standhalten. Wesentliche Auslegungsmerkmale für die Fieldrohre sind deren Innendurchmesser, Wandstärke, Anzahl und Länge. Deren konstruktive Ausgestaltung erfolgt in Kenntnis der Reaktionsbedingungen und der bekannten Werte für die Exothermie und Kinetik der Hydroformylierungsreaktion nach dem Fachmann geläufigem, ingenieurtechnischem Wissen.

Als weiteres erfindungsgemäßes Auslegungsmerkmal für die Fieldrohre ist vorgegeben, dass die von denselben belegte Querschnittsfläche, bezogen auf die Querschnittsfläche des Einsteckrohres im Bereich von 0,03 m²/m² bis 0,30 m²/m² liegt. Bevorzugt liegt die von den Fieldrohren belegte Querschnittsfläche bezogen auf die Querschnittsfläche des Einsteckrohres im Bereich von 0,07 m²/m² bis 0,25 m²/m². Bei der Angabe der Querschnittsfläche handelt es sich um die Gesamtquerschnittsfläche des Einsteckrohres, d. h. die Wandstärke des Einsteckrohres wird nicht abgezogen.

Während es einerseits erforderlich ist, über die Fieldrohre so viel Wärmeübertragungsfläche einzubringen, dass die Reaktionswärme der stark exothermen Hydroformylierung effektiv abgeführt wird, wird durch die obige Bedingung sichergestellt, dass die Anzahl an Fieldrohren im Einsteckrohr nicht so hoch ist, dass es zu einer fluiddynamischen Einschnürung (Käfigeffekt) kommt, die zu einer ungleichmäßigen Geschwindigkeitsverteilung der Flüssigkeit im Einsteckrohr führen würde. Dies würde eine Ungleichverteilung des Gasanteils in der Flüssigkeit bewirken und könnte die interne Umlaufströmung (Zirkulationsströmung) dahingehend beeinflussen, dass der Volumenstrom kleiner wird bzw. sich eine Rückströmung im Randbereich des Einsteckrohres ausbildet. Dies würde wiederum dazu führen, dass weniger Gasblasen mitgerissen werden und somit der Kohlenmonoxidpartialdruck, insbesondere im Ringraum außerhalb des Einsteckrohres, so niedrige Werte erreichen kann, dass es zum unerwünschten Ausfall von metallischem Cobalt kommt.

Bevorzugt beträgt die Querschnittsfläche des Einsteckrohres bezogen auf die Querschnittsfläche des Reaktors (1) 0,60 m²/m² bis 0,75 m²/m², besonders bevorzugt 0,66 m²/m² bis 0,72 m²/m². Bei der Angabe der Querschnittsflächen handelt es sich um die Gesamtquerschnittsfläche des Einsteckrohres (d. h. einschließlich der Wandstärke) und um die Querschnittsfläche des Reaktors, die sich aus seinem Innendurchmesser ergibt (d. h. ausschließlich der Wandstärke).

Die Fieldrohre sind im oberen Bereich des Reaktors befestigt. Die Befestigung kann in üblicher Weise, beispielsweise durch Schweißen, erfolgen. Die Befestigung erfolgt dabei z. B. am Deckel des Reaktors. Die Vorrichtungen für die Zuführung und Abführung des Kühlmittels, das durch die Fieldrohre strömt, sind üblicherweise in den Reaktorkopf integriert. Dazu kann der Reaktorkopf beispielsweise zwei separate Kompartimente aufweisen, wobei eines mit dem Einlass für das Kühlmittel versehen ist und von dem aus das Kühlmittel in die Fieldrohre verteilt wird und das andere das aus den Fieldrohren ausströmende erwärmte Kühlmittel aufnimmt, welches dann durch einen Auslass den Reaktorkopf verlässt. Die verschiedenen Teile, die den Reaktorkopf bilden, können durch Schweißen, adhäsive Verbindungen, Nieten, und Verschrauben miteinander verbunden sein. In einer bevorzugten Ausführung sind die verschiedenen Teile, die den Reaktorkopf bilden, durch Verschrauben miteinander verbunden.

Um die Fieldrohre im Innenraum des Reaktors in der gewünschten geometrischen Anordnung zu halten, können weitere Haltevorrichtungen vorgesehen sein. Im Bereich des Einsteckrohres sind diese Haltevorrichtungen z. B. an der Innenwandung des Einsteckrohres angebracht. Geeignete Haltevorrichtungen sind insbesondere Gitter, durch die die Fieldrohre geführt werden. Diese Haltevorrichtungen weisen nicht mit Fieldrohren belegte freie Querschnittsflächen für das strömende Reaktionsmedium auf. Eine oder falls vorhandenen mehrere oder alle vorhandenen Haltevorrichtungen können zusätzliche Strömungseinbauten aufweisen, die die Funktion von Mischelementen übernehmen können. So kann eine mit Mischelementen versehene Haltevorrichtung gleichzeitig als statischer Mischer wirken. Beispielsweise kann eine Haltevorrichtung in Form eines Gitters, durch das die Fieldrohre geführt werden, zusätzliche Stege aufweisen und so zusätzlich die Funktion eines statischen Mischers übernehmen.

In einer bevorzugten Ausführung weist der erfindungsgemäße zylindrische Reaktor mehrere Haltevorrichtungen auf. Bevorzugt ist in der unteren Hälfte des Reaktors mindestens eine Haltevorrichtung als statischer Mischer ausgestaltet. In einer besonders bevorzugten Ausgestaltung ist die unterste (am nächsten an der/den Einlassdüse(n) gelegene) Haltevorrichtung als statischer Mischer ausgestaltet.

Da die Fieldrohre von einem Kühlmittel durchströmt sind, können sie eine deutliche Temperaturdifferenz zum Reaktionsmedium und der/den Haltevorrichtung(en) aufweisen. Auch sind Temperaturschwankungen des Kühlmittels und des Reaktionsmediums nicht ausgeschlossen. Daher werden die Fieldrohre beweglich, d. h. nicht fest eingespannt sondern über ein oder mehrere Auflager, in der Haltevorrichtung angeordnet. Damit lassen sich Materialspannungen und daraus resultierende Schäden durch unterschiedliche Temperaturausdehnung vermeiden.

Zur Einschränkung der Beweglichkeit können die Fieldrohre oberhalb und/oder unterhalb einer Haltevorrichtung Verdickungen an ihren Außenwandungen aufweisen. Die Verdickungen werden vorzugsweise gleichmäßig über den Umfang der Außenwandung auf jeweils gleicher Höhe verteilt. Besonders bevorzugt werden die Verdickungen jeweils paarweise oberhalb und unterhalb einer Haltevorrichtung angeordnet. Beispielsweise werden zwei, drei, vier, fünf oder sechs Paare entlang des Umfangs verteilt. Diese Verdickungen werden vorzugsweise von außen an der Außenwandung der Fieldrohre mittels einer stoffschlüssigen Verbindung angebracht, beispielsweise durch Schweißen, Löten, Kleben. Des Weiteren kann die Haltevorrichtung auf den Verdickungen der Fieldrohre aufliegen. Da die Haltevorrichtung an der Innenwandung des Einsteckrohres befestigt ist, können die Verdickungen somit auch zur Fixierung des Einsteckrohres dienen.

Die Befestigung der Haltevorrichtungen an der Innenwandung des Einsteckrohres kann in üblicher Weise mittels einer stoffschlüssigen Verbindung, beispielsweise durch Schweißen, Löten, Kleben, insbesondere durch Schweißen, erfolgen.

Um das Einstecksteckrohr im Innenraum des Reaktors in der gewünschten geometrischen Anordnung zu halten, sind weitere Führungsvorrichtungen vorgesehen. Die Führung des Einsteckrohres an der Innenwandung des zylindrischen Reaktors kann durch Abstandshalter oder Rollen im Ringraum erfolgen. Dies ermöglicht es, temperaturbedingte Dimensionsänderungen zu kompensieren.

Die Führungsvorrichtungen können an der Außenwand des Einsteckrohres oder an der Reaktorinnenwand befestigt sein. Bevorzugt sind sie am Einsteckrohr außen befestigt. Sie werden entsprechend der Rohranordnung über den Umfang des Ringraumes verteilt. Bevorzugt werden die Führungsvorrichtungen gleichmäßig über den Umfang des Ringraumes verteilt. Sie bilden beispielsweise ein gleichseitiges Dreieck, ein gleichmäßiges Sechseck, ein Quadrat, ein gleichmäßiges Achteck. Die Führungsvorrichtungen sind bevorzugt auf Höhe einer Haltevorrichtung angeordnet.

Der erfindungsgemäße Reaktor weist weiterhin bevorzugt einen Gasverteiler auf, der besonders bevorzugt als Gasverteilerring ausgebildet ist. In einer bevorzugten Ausgestaltung weist der erfindungsgemäße Reaktor einen Gasverteilerring am unteren Ende des Einsteckrohres, insbesondere an seiner Innenwand auf. Über den Gasverteilerring kann dem Reaktor ein Teilstrom der gasförmigen Komponente des Eduktgemischs zugeführt werden. Wird der erfindungsgemäße Reaktor zur Hydroformylierung eingesetzt, so kann diesem über den Gasverteilerring ein Teilstrom des Synthesegases zugeführt werden. Falls ein solcher Teilstrom eingesetzt wird, beträgt der Teilstrom bevorzugt 1 bis 20 %, besonders bevorzugt 2 bis 15 % des gesamten, dem Reaktor zugeführten Gasstromes.

Indem ein zusätzlicher Gasverteiler vorgesehen ist, wird eine deutlich verbesserte radiale Verteilung von Gas und Flüssigkeit und somit ein deutlich vergrößerter interner Zirkulationsstrom erreicht. Dadurch entsteht entlang der Innenwand des Einsteckrohres eine von unten nach oben gerichtete Strömung, die die Schlaufenströmung um das Einsteckrohr unterstützt.

Der erfindungsgemäße Reaktor weist bevorzugt einen statischen Mischer oder mehrere statische Mischer auf, die im Einsteckrohr angeordnet sind. Dadurch wird die radiale Verteilung des Reaktionsgemisches im Einsteckrohr verbessert. Wie oben beschrieben, können die Haltevorrichtungen zusätzlich die Funktion eines statischen Mischers übernehmen. Bevorzugt werden einzelne der vorhandenen Haltegitter der Fieldrohre als statische Mischer genutzt, in denen die Anzahl der Stege, die zum Halten der Fieldrohre verwendet werden, maximiert, das heißt möglichst groß gewählt wird. Dadurch ist der freie Querschnitt der so gestalteten Haltegitter geringer. Die Erhöhung des Druckverlustes führt zu einer zusätzlichen Verwirbelung und damit zu einer besseren Gas- und Flüssigkeitsverteilung. Durch dieses gezielte Erzeugen von Druckverlust und Verwirbelung im Einsteckrohr wird zusätzlich Energie dissipiert.

Das an beiden Enden offene Einsteckrohr, das die Fieldrohre umhüllt und an seinem unteren Ende über diese hinausragt, wird bevorzugt so weit wie möglich an das obere Ende des Reaktors geführt, damit Totzonen im oberen Bereich weitestgehend vermieden werden. Dabei sollte jedoch vermieden werden, dass der Druckverlust zu hoch wird. Es hat sich gezeigt, dass es besonders vorteilhaft ist, wenn das Einsteckrohr die Fieldrohre bis auf 80 % der Länge derselben, bevorzugt bis auf 90 % der Länge derselben umhüllt.

Eine weitere Verbesserung der Durchmischung kann erreicht werden, indem oberhalb des Einsteckrohres eine senkrecht dazu angeordnete Prallplatte vorgesehen ist, durch die die Fieldrohre hindurchgeführt werden. Die Prallplatte ist bevorzugt scheibenförmig, und weist einen Durchmesser von mindestens dem halben Durchmesser des Einsteckrohres und weniger als dem Innendurchmesser des Reaktors auf. Die Dicke der Prallplatte ist so zu wählen, dass sie mechanisch dem Strömungsdruck standhält. Bevorzugt liegt die Dicke der Prallplatte in einem Bereich von 5 bis 10 mm.

Eine weitere Verbesserung der Durchmischung im erfindungsgemäßen Reaktor kann erreicht werden, indem die Reaktorenden gewölbt ausgebildet werden. Es ist insbesondere vorteilhaft, den Sumpf des Reaktors gewölbt auszubilden. Hierbei ist es besonders bevorzugt, wenn der Boden innen gewölbt ausgebildet ist.

Die Abführung des Reaktionsgemischs erfolgt zweiphasig durch den Deckel des Reaktors. Wird eine Prallplatte verwendet, erfolgt die Abführung des Reaktionsgemischs oberhalb der Prallplatte.

Die Aufarbeitung des flüssigen Reaktionsaustrags, der mindestens einen Aldehyd als Hydroformylierungsprodukt, nicht umgesetzte Olefine, gelöstes Synthesegas, den homögen gelösten Hydroformylierungskatalysator, sowie gegebenenfalls Nebenprodukte der Hydroformylierungsreaktion enthält, erfolgt nach üblichen, dem Fachmann bekannten Verfahren. So beschreibt z. B. die WO 01/14297 ein kontinuierliches Verfahren zur Hydroformylierung von Olefinen mit 6 bis 20 Kohlenstoffatomen. Darin wird ausführlich beschrieben, wie ein Reaktionsaustrag der Hydroformylierung mit einem unmodifizierten Cobaltkatalysator aufgearbeitet und speziell wie der Cobaltkatalysator im Wesentlichen ohne Katalysatorverluste wieder in die Reaktion zurückgeführt werden kann.

Die Erfindung wird im Folgenden anhand der Figuren und Ausführungsbeispiele näher erläutert.

### FIGURENBESCHREIBUNG

- Figur 1: zeigt einen Längsschnitt durch eine bevorzugte Ausführungsform eines erfindungsgemäßen Reaktors.
- Figur 2: zeigt einen Längsschnitt durch ein einzelnes Fieldrohr.
- Figur 2a: zeigt eine Detailansicht eines einzelnen Fieldrohres in einer Haltevorrichtung mit oberhalb und unterhalb der Haltevorrichtung an der Außenwand der Fieldrohres angebrachten Verdickungen.
- Figur 3: zeigt schematisch die Flüssigkeitsströmung durch die in Figur 1 dargestellte Ausführungsform.
- Figur 4: zeigt eine Detailzeichnung des unteren Reaktorbereiches in der Ausgestaltung mit einem Gasverteilerring.
- Figur 5: zeigt einen Querschnitt durch einen in Figur 1 dargestellten Reaktor.

In den Figuren bezeichnen gleiche Bezugszeichen jeweils gleiche oder entsprechende Merkmale.
- 1: Reaktor
- 2: Fieldrohre
- 3: Einsteckrohr
- 4: Einlassdüse
- 5: Gasverteilerring
- 6: Haltevorrichtung
- 6a: Haltevorrichtung und statischer Mischer
- 7: Prallplatte
- 8: Reaktormantel
- 9: Ringraum
- 10: Außenrohr
- 11: Innenrohr
- 12: Reaktorkopf
- 13: Hebeösen
- 14: Verdickungen
- 15: Abstandshalter

Der Längsschnitt in Figur 1 zeigt schematisch einen bevorzugten zylindrischen Reaktor 1 mit vertikaler Längsachse mit einer Vielzahl von Fieldrohren 2 und einem schematisch dargestelltem Reaktorkopf 12. Die Fieldrohre 2 sind an einem Rohrboden am oberen Ende des Reaktors 1, der vorliegend das untere Ende des Reaktorkopfes 12 bildet, eingeschweißt. Koaxial im Reaktor 1 ist ein an beiden Enden offenes Einsteckrohr 3 angeordnet, das die Fieldrohre 2 umhüllt und an seinem unteren Ende über diese hinausragt. Der Reaktorkopf enthält (nicht dargestellt) unter anderem Teile des Kühlmittelkreislaufes zur Verteilung des einströmenden kalten Kühlmittels auf die einzelnen Fieldrohre 2 und zur Abführung des aus den einzelnen Fieldrohren 2 austretenden erwärmten Kühlmittels und des entstandenen Dampfes. Der Reaktorkopf 12 enthält weiterhin den Auslass für das Reaktionsgemisch, wobei ein gemeinsamer Austrag von gasförmigen und flüssigen Komponenten erfolgen kann. Die Zuführung der Edukte erfolgt über eine Einlassdüse 4 am unteren Ende des Reaktors 1. Die Einlassdüse 4 ist als Ejektorstrahldüse ausgestaltet über die gleichzeitig die C₆-C₂₀-Olefine und das Synthesegas gleichzeitig zugeführt werden. Falls gewünscht, kann zusätzlich wässriger Übergangsmetallkatalysator, insbesondere der Metall-Carbonylkomplexkatalysator, der Düse 4 mit einem der Ströme zugeführt werden. Über einen Gasverteilerring 5 am unteren Ende des Einsteckrohres 3 kann ein Teilstrom des Synthesegases zugeführt werden. Beispielhaft sind eine als statischer Mischer 6a ausgestaltete Haltevorrichtung und zwei weitere Haltevorrichtungen 6 im Einsteckrohr 3 dargestellt. Oberhalb des Einsteckrohres 3 befindet sich eine senkrecht zum Einsteckrohr 3 angeordnete Prallplatte 7.

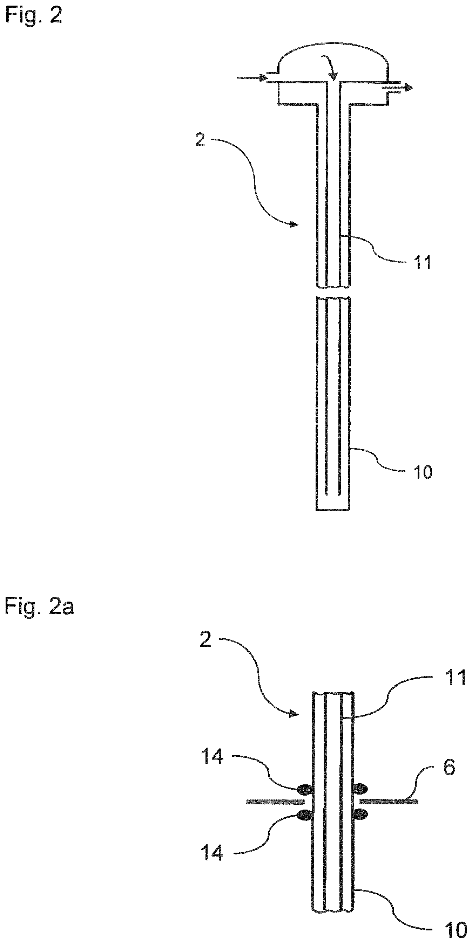

Figur 2 zeigt beispielhaft schematisch ein einzelnes Fieldrohr 2, das jeweils aus einer Hülse, hier und im Folgenden als Außenrohr 10 bezeichnet, und einem koaxialen Innenrohr 11 besteht. Das Außenrohr 10 ist an seinem unteren Ende geschlossen, während das koaxiale Innenrohr 11 an seinem unteren Ende offen ist. In der dargestellten Ausführungsform kann Kühlmittel, wie beispielsweise Wasser, insbesondere Kesselspeisewasser, von oben in das Innenrohr 11 eingeführt und am oberen Ende des Au-ßenrohres 10 als erwärmtes Kühlmittel, beispielsweise als Dampf-Flüssigkeitsmischung abgezogen werden. Das Kühlmittel wird üblicherweise durch das Innenrohr nach unten und zwischen Innenrohr 11 und Außenrohr 10 nach oben geführt. Alternativ kann der Kühlmittelfluss aber auch umgekehrt werden. Zuführung und Abführung des Kühlmittels, das durch die Fieldrohre 2 strömt, sind üblicherweise in den Reaktorkopf 12 integriert. Dazu kann der Reaktorkopf 12 beispielsweise zwei separate Kompartimente aufweisen, wobei eines mit dem Einlass für das Kühlmittel versehen ist und von dem aus das Kühlmittel in die Fieldrohre 2 verteilt wird und das andere das aus den Fieldrohren 2 ausströmende erwärmte Kühlmittel aufnimmt, welches dann durch einen Auslass den Reaktorkopf 12 verlässt.

Figur 2a zeigt eine Detailansicht eines einzelnen Fieldrohres 2 in einer Haltevorrichtung 6. Oberhalb und unterhalb der Haltevorrichtung 6 sind an der Außenwand des Fieldrohres 2 Verdickungen angebracht, die ein Auflager für die Haltevorrichtung 6 bilden.

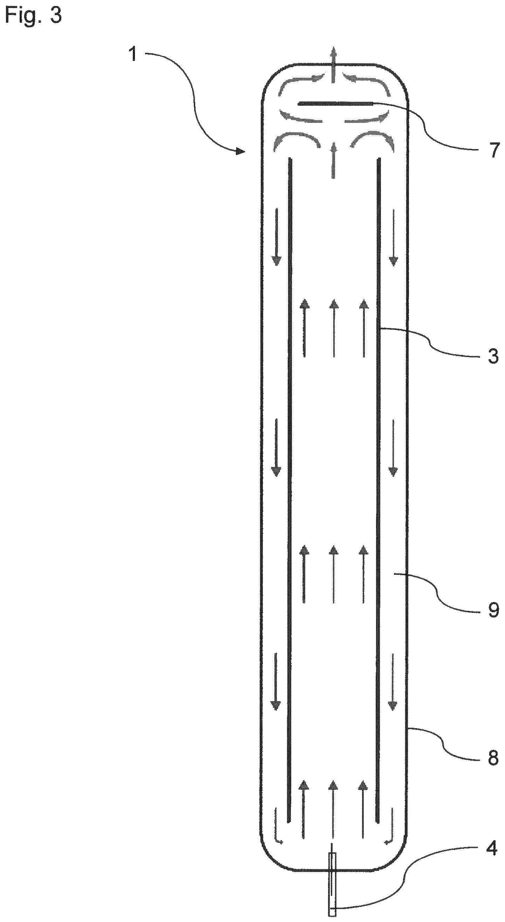

Figur 3 veranschaulicht die Flüssigkeitsströmung in dem in Figur 1 dargestellten Reaktor 1. Die Flüssigkeitsströmung wird dabei schematisch durch Pfeile dargestellt. Innerhalb des Einsteckrohres 3 wird eine Aufwärtsströmung durch die Strahldüse 4 induziert. Durch die Prallplatte 7 erfolgt eine Umlenkung der Strömung zu einer Abwärtsströmung im Ringraum 9 zwischen der Reaktorinnenwand und dem Einsteckrohr 3.

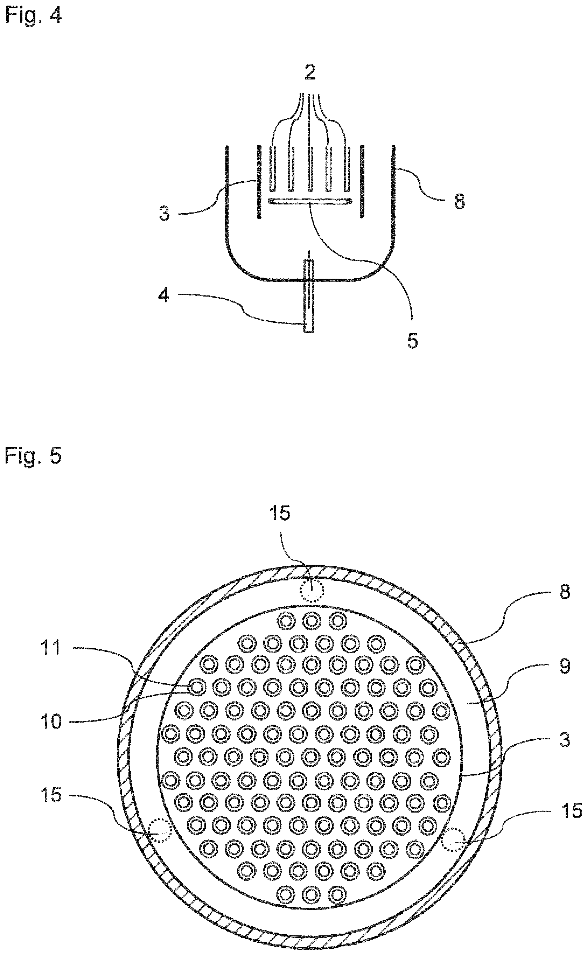

Figur 4 zeigt eine Detailzeichnung des unteren Reaktorbereichs einer erfindungsgemäßen Ausführungsform. Danach weist der Reaktor 1 eine Strahldüse 4 zum Eindüsen des Eduktgemisches auf. Über einen Gasverteilerring 5 am unteren Ende des Einsteckrohres 3 kann dem Reaktor 1 ein Teilstrom des Synthesegases zugeführt werden.

Figur 5 zeigt einen Querschnitt durch den in Figur 1 dargestellten Reaktor an einer Stelle, an der sich keine Haltevorrichtung befindet. Dieser Querschnitt wird hier und im Folgenden auch als Rohrspiegel bezeichnet. Der Rohrspiegel lässt Anzahl, relative Abmessungen und Anordnung der jeweils aus Außenrohr 10 und koaxialen Innenrohr 11 bestehenden Fieldrohre innerhalb des Einsteckrohres 3 erkennen. Dargestellt ist eine mögliche Anordnung der Fieldrohre im Einsteckrohr 3 in Form von gleichseitigen Dreiecken, auch als versetzte Rohranordnung bezeichnet. Ebenfalls geeignet wäre eine Anordnung der Fieldrohre im Einsteckrohr in quadratischer Form, auch als fluchtende Rohranordnung bezeichnet.

Das Einsteckrohr 3 ist koaxial zum Reaktormantel 8 angeordnet und umhüllt die jeweils aus Außenrohr 10 und koaxialen Innenrohr 11 bestehenden Fieldrohre. Zwischen Reaktormantel 8 und Einsteckrohr befindet sich ein Ringraum 9. Die Abstandshalter 15 sind entsprechend der Rohranordnung über den Umfang des Ringraumes 9 verteilt. Im vorliegenden Fall bilden sie ein gleichseitiges Dreieck; geeignet wäre aber auch eine Anordnung als gleichmäßiges Sechseck oder im Fall einer fluchtenden Anordnung als Quadrat. Die Abstandshalter 15 sollen hier nur angedeutet sein, da sie bevorzugt auf Höhe einer Haltevorrichtung angeordnet sind.

### BEISPIELE

### Beispiel 1

Es wird eine Hydroformylierungsanlage eingesetzt, die einen Hauptreaktor und einen Nachreaktor umfasst. Der Hauptreaktor hat eine Länge von 18,0 m und einen inneren Durchmesser von 1,0 m. Der obere Rohrboden, der gleichzeitig der Deckel des Reaktors ist, ist mit 64 Fieldrohren mit einem Außendurchmesser von 30,0 mm bestückt. Im Hauptreaktor ist ein Einsteckrohr mit einem Innendurchmesser von 0,84 m angeordnet. Die Edukte (Isoocten und Synthesegas mit 40 vol-% Kohlenmonoxid und 60 vol-% Wasserstoff) und die wässrige Cobalt-Acetatlösung werden über eine Düse am Boden des Reaktors eingebracht. Der Hauptreaktor wird bei 187 °C und 275 bar betrieben. Die Temperatur im Nachreaktor liegt auf gleichem Niveau, der Druck wird um 3 bar unterhalb des Druckes im Hauptreaktor gehalten. Es wurden 1 t/h Synthesegas und 3 t/h Isoocten, hergestellt nach WO 95/14647, eingesetzt.

Das Verhältnis der Gesamtwärmeübertragungsfläche der Fieldrohre bezogen auf das Innenvolumen des Hauptreaktors liegt bei 9,4 m²/m³. Die von den Fieldrohren belegte Querschnittsfläche bezogen auf die Querschnittsfläche des Einsteckrohres liegt bei 0,08 m²/m².

In dieser Hydroformylierungsanlage wird Isononanol hergestellt. Um die Verluste durch im Reaktor abgelagertes Cobalt auszugleichen, müssen innerhalb eines Jahres 160 kg Cobalt als Cobaltacetatlösung ergänzt werden. Dies entspricht 0,01 kg Cobalt je Tonne Produkt.

Dieser Reaktor konnte fünf Jahre betrieben werden, bis die Cobaltablagerungen durch den Einsatz von verdünnter Salpetersäure entfernt wurden (siehe internationale Patentanmeldung WO2015/018 710).

### Vergleichsbeispiel

Die Anlage und die Betriebsbedingungen entsprechen dem erfindungsgemäßen Beispiel 1. Abweichend hiervon ist jedoch der Hauptreaktor mit 158 Fieldrohen mit einem äußeren Durchmesser von 48,3 mm bestückt und das Einsteckrohr hat einen Innendurchmesser von 1,23 m.

Das Verhältnis der Gesamtwärmeübertragungsfläche der Fieldrohre bezogen auf das Innenvolumen des Reaktors liegt bei 16,1 m²/m³. Die von den Fieldrohren belegte Querschnittsfläche bezogen auf die Querschnittsfläche des Einsteckrohres liegt bei 0,25 m²/m².

In dieser Hydroformylierungsanlage wird ebenfalls Isononanol aus Isoocten und Synthesegas mit 40 vol-% Kohlenmonoxid und 60 vol-% Wasserstoff mit Hilfe eines Cobaltkatalysators hergestellt. Der Hauptreaktor wird bei 187 °C und 275 bar betrieben. Die Temperatur im Nachreaktor liegt auf gleichem Niveau, der Druck wird um 3 bar unterhalb des Druckes im Hauptreaktor gehalten. Es wurden 1 t/h Synthesegas und 3 t/h Isoocten, hergestellt nach WO 95/14647, eingesetzt.

Innerhalb eines Jahres müssen 5000 kg Cobalt als Cobaltacetatlösung ergänzt werden, um die Verluste durch im Reaktor abgelagertes Cobalt auszugleichen. Dies entspricht 0,06 kg Cobalt je Tonne Produkt, die Cobaltverluste sind somit deutlich höher gegenüber dem erfindungsgemäßen Beispiel.

Es kam nach etwa einem Jahr zu Verstopfungen in den Rohrleitungen und die Cobaltablagerungen mussten durch den Einsatz von verdünnter Salpetersäure entfernt werden, um die Verstopfung zu beseitigen.

## Patentansprüche

1. Zylindrischer Reaktor (1) mit vertikaler Längsachse
- mit einer Vielzahl von Fieldrohren (2), die parallel zur Längsachse des Reaktor (1) ausgerichtet und am oberen Ende des Reaktors (1) befestigt sind,
- mit einem an beiden Enden offenen Einsteckrohr (3), das die Fieldrohre (2) umhüllt und an seinem unteren Ende über dieselben hinausragt,
- mit einer Einlassdüse (4) oder mehreren Einlassdüsen (4) am Boden des Reaktors (1) zum Eindüsen eines Eduktgemisches, das wenigstens eine gasförmige und wenigstens eine flüssige Komponente enthält, wobei
- die Fieldrohre (2) in ihrer Anzahl und ihren Abmessungen dergestalt ausgelegt sind, dass
- die Gesamtwärmeübertragungsfläche derselben bezogen auf das Innenvolumen des Reaktors im Bereich von 1 m²/m³ bis 12 m²/m³ liegt und
- die von den Fieldrohren (2) belegte Querschnittsfläche bezogen auf die Querschnittsfläche des Einsteckrohres (3) im Bereich von 0,03 m²/m² bis 0,30 m²/m² liegt.

2. Zylindrischer Reaktor (1) nach Anspruch 1, **dadurch gekennzeichnet, dass** die Querschnittsfläche des Einsteckrohres (3) bezogen auf die Querschnittsfläche des Reaktors (1) 0,60 m²/m² bis 0,75 m²/m², bevorzugt 0,66 m²/m² bis 0,72 m²/m², beträgt.

3. Zylindrischer Reaktor (1) nach Anspruch 1 oder 2, **dadurch gekennzeichnet, dass** er einen Gasverteilerring (5) am unteren Ende des Einsteckrohres (3), an der Innenwand desselben, aufweist, über den ein Teilstrom der gasförmigen Komponente des Eduktgemischs zugeführt wird.

4. Zylindrischer Reaktor (1) nach einem der vorhergehenden Ansprüche, **dadurch gekennzeichnet, dass** er eine Haltevorrichtung (6) oder mehrere Haltevorrichtungen (6) im Einsteckrohr (3) aufweist.

5. Zylindrischer Reaktor (1) nach einem der vorhergehenden Ansprüche, **dadurch gekennzeichnet, dass** er einen statischen Mischer (6a) oder mehrere statische Mischer (6a) im Einsteckrohr (3) aufweist.

6. Zylindrischer Reaktor (1) nach Anspruch 4 oder 5, **dadurch gekennzeichnet, dass** eine oder mehrere der Haltevorrichtungen (6) zusätzlich als statische Mischer wirken.

7. Zylindrischer Reaktor (1) nach einem der vorhergehenden Ansprüche, **dadurch gekennzeichnet, dass** die Fieldrohre (2) in ihrer Anzahl und ihren Abmessungen dergestalt ausgelegt sind, dass
- die Gesamtwärmeübertragungsfläche derselben bezogen auf das Innenvolumen des Reaktors im Bereich von 7 m²/m³ bis 11 m²/m³ liegt und
- die von den Fieldrohren (2) belegte Querschnittsfläche bezogen auf die Querschnittsfläche des Einsteckrohres (3) im Bereich von 0,07 m²/m² bis 0,25 m²/m² liegt.

8. Zylindrischer Reaktor (1) nach einem der vorhergehenden Ansprüche, **dadurch gekennzeichnet, dass** das Einsteckrohr (3) die Fieldrohre (2) bis auf 80 % der Länge derselben, bevorzugt bis auf 90 % der Länge derselben, umhüllt.

9. Zylindrischer Reaktor (1) nach einem der vorhergehenden Ansprüche, **dadurch gekennzeichnet, dass** er oberhalb des Einsteckrohres (3) eine Prallplatte (7) aufweist.

10. Kontinuierliches Verfahren zur Herstellung von C₇-C₂₁-Oxoprodukten durch Hydroformylierung wenigstens eines C₆-C₂₀-Olefins mit Synthesegas in Gegenwart eines homogen gelösten Übergangsmetallkatalysators, **dadurch gekennzeichnet, dass** das Verfahren in einem zylindrischen Reaktor (1) mit vertikaler Längsachse durchgeführt wird,
- mit einer Vielzahl von Fieldrohren (2), die parallel zur Längsachse des Reaktor (1) ausgerichtet und in einem Rohrboden am oberen Ende des Reaktors (1) eingeschweißt sind,
- mit einem an beiden Enden offenen Einsteckrohr (3), das die Fieldrohre (2) umhüllt und an seinem unteren Ende über dieselben hinausragt,
- mit einer Einlassdüse (4) oder mehreren Einlassdüsen (4) am Boden des Reaktors (1) zum Eindüsen eines Eduktgemisches, enthaltend das C₆-C₂₀-Olefin, das Synthesegas und gegebenenfalls den Übergangskatalysator, wobei
- die Fieldrohre (2) in ihrer Anzahl und ihren Abmessungen dergestalt ausgelegt sind, dass
- die Gesamtwärmeübertragungsfläche derselben bezogen auf das Innenvolumen des Reaktors im Bereich von 1 m²/m³ bis 12 m²/m³ liegt und
- die von den Fieldrohren (2) belegte Querschnittsfläche bezogen auf die Querschnittsfläche des Einsteckrohres (3) im Bereich von 0,03 m²/m² bis 0,30 m²/m² liegt.

11. Verfahren nach Anspruch 10, **dadurch gekennzeichnet, dass** der Übergangsmetallkatalysator ein Metall-Carbonylkomplexkatalysator, vorzugsweise ein Cobalt-Carbonylkomplexkatalysator ist.

## Claims

1. A cylindrical reactor (1) having a vertical longitudinal axis,
- having a multiplicity of Field tubes (2) which are oriented parallel to the longitudinal axis of the reactor (1) and affixed to the upper end of the reactor (1),
- having a tube insert (3) open at both ends which envelops the Field tubes (2) and at its lower end projects beyond said tubes,
- having an inlet nozzle (4) or a plurality of inlet nozzles (4) at the bottom of the reactor (1) for injecting a reactant mixture comprising at least one gaseous component and at least one liquid component, wherein
- the Field tubes (2) are configured in terms of their number and their dimensions such that
- the total heat transfer area of said tubes per unit internal volume of the reactor is in the range from 1 m²/m³ to 12 m²/m³ and
- the cross sectional area occupied by the Field tubes (2) per unit cross sectional area of the tube insert (3) is in the range from 0.03 m²/m² to 0.30 m²/m².

2. The cylindrical reactor (1) according to claim 1, wherein the cross sectional area of the tube insert (3) per unit cross sectional area of the reactor (1) is 0.60 m²/m² to 0.75 m²/m², preferably 0.66 m²/m² to 0.72 m²/m².

3. The cylindrical reactor (1) according to claim 1 or 2, wherein said reactor comprises a gas distributor ring (5) at the lower end of the tube insert (3) at the inner wall thereof via which a substream of the gaseous component of the reactant mixture is fed.

4. The cylindrical reactor (1) according to any of the preceding claims, wherein said reactor comprises a holding device (6) or a plurality of holding devices (6) in the tube insert (3).

5. The cylindrical reactor (1) according to any of the preceding claims, wherein said reactor comprises a static mixer (6a) or a plurality of static mixers (6a) in the tube insert (3).

6. The cylindrical reactor (1) according to claim 4 or 5, wherein one or more of the holding devices (6) additionally function as static mixers.

7. The cylindrical reactor (1) according to any of the preceding claims, wherein the Field tubes (2) are configured in terms of their number and their dimensions such that
- the total heat transfer area of said tubes per unit internal volume of the reactor is in the range from 7 m²/m³ to 11 m²/m³ and
- the cross sectional area occupied by the Field tubes (2) per unit cross sectional area of the tube insert (3) is in the range from 0.07 m²/m² to 0.25 m²/m².

8. The cylindrical reactor (1) according to any of the preceding claims, wherein the tube insert (3) envelops the Field tubes (2) to an extent of 80% of the length thereof, preferably to an extent of 90% of the length thereof.

9. The cylindrical reactor (1) according to any of the preceding claims, wherein said reactor comprises a baffle plate (7) above the tube insert (3).

10. A continuous process for producing C₇-C₂₁-oxo products by hydroformylation of at least one C₆-C₂₀-olefin with synthesis gas in the presence of a homogeneously dissolved transition metal catalyst, wherein the process is carried out in a cylindrical reactor (1) having a vertical longitudinal axis,
- having a multiplicity of Field tubes (2) which are oriented parallel to the longitudinal axis of the reactor (1) and welded into a tube plate at the upper end of the reactor (1),
- having a tube insert (3) open at both ends which envelops the Field tubes (2) and at its lower end projects beyond said tubes,
- having an inlet nozzle (4) or a plurality of inlet nozzles (4) at the bottom of the reactor (1) for injecting a reactant mixture comprising the C₆-C₂₀-olefin, the synthesis gas and optionally the transition catalyst, wherein
- the Field tubes (2) are configured in terms of their number and their dimensions such that
- the total heat transfer area of said tubes per unit internal volume of the reactor is in the range from 1 m²/m³ to 12 m²/m³ and
- the cross sectional area occupied by the Field tubes (2) per unit cross sectional area of the tube insert (3) is in the range from 0.03 m²/m² to 0.30 m²/m².

11. The process according to claim 10, wherein the transition metal catalyst is a metal carbonyl complex catalyst, preferably a cobalt carbonyl complex catalyst.

## Revendications

1. Réacteur cylindrique (1) avec un axe longitudinal vertical
- avec une multiplicité de tubes Field (2), qui sont orientés parallèlement à l'axe longitudinal du réacteur (1) et qui sont fixés à l'extrémité supérieure du réacteur (1),
- avec un tube de fourreau (3) ouvert aux deux extrémités, qui entoure les tubes Field (2) et qui dépasse ceux-ci à son extrémité inférieure,
- avec une buse d'admission (4) ou plusieurs buses d'admission (4) au fond du réacteur (1) pour injecter un mélange d'éduit, qui contient au moins un composant gazeux et au moins un composant liquide, dans lequel
- les tubes Field (2) sont conçus, en nombre et en dimensions, de telle manière que
- la surface totale de transfert de chaleur de ceux-ci rapportée au volume intérieur du réacteur se situe dans la plage de 1 m²/m³ à 12 m²/m³, et
- la surface de section transversale occupée par les tubes Field (2) rapportée à la surface de section transversale du tube de fourreau (3) se situe dans la plage de 0,03 m²/m² à 0,30 m²/m².

2. Réacteur cylindrique (1) selon la revendication 1, **caractérisé en ce que** la surface de section transversale du tube de fourreau (3) rapportée à la surface de section transversale du réacteur (1) vaut 0,60 m²/m² à 0,75 m²/m², de préférence 0,66 m²/m² à 0,72 m²/m².

3. Réacteur cylindrique (1) selon une revendication 1 ou 2, **caractérisé en ce qu'**il présente un anneau de répartition de gaz (5) à l'extrémité inférieure du tube de fourreau (3), à la paroi intérieure de celui-ci, par lequel on fournit un flux partiel du composant gazeux du mélange d'éduit.

4. Réacteur cylindrique (1) selon l'une quelconque des revendications précédentes, **caractérisé en ce qu'**il présente un dispositif de maintien (6) ou plusieurs dispositifs de maintien (6) dans le tube de fourreau (3).

5. Réacteur cylindrique (1) selon l'une quelconque des revendications précédentes, **caractérisé en ce qu'**il présente un mélangeur statique (6a) ou plusieurs mélangeurs statiques (6a) dans le tube de fourreau (3).

6. Réacteur cylindrique (1) selon une revendication 4 ou 5, **caractérisé en ce qu'**un ou plusieurs des dispositifs de maintien (6) agit/agissent en outre comme mélangeur(s) statique(s).

7. Réacteur cylindrique (1) selon l'une quelconque des revendications précédentes, **caractérisé en ce que** les tubes Field (2) sont conçus, en nombre et en dimensions, de telle manière que
- la surface totale de transfert de chaleur de ceux-ci rapportée au volume intérieur du réacteur se situe dans la plage de 7 m²/m³ à 11 m²/m³, et
- la surface de section transversale occupée par les tubes Field (2) rapportée à la surface de section transversale du tube de fourreau (3) se situe dans la plage de 0,07 m²/m² à 0,25 m²/m².

8. Réacteur cylindrique (1) selon l'une quelconque des revendications précédentes, **caractérisé en ce que** le tube de fourreau (3) entoure les tubes Field (2) jusque sur 80 % de leur longueur, de préférence jusque sur 90 % de leur longueur.

9. Réacteur cylindrique (1) selon l'une quelconque des revendications précédentes, **caractérisé en ce qu'**il présente une plaque d'impact (7) au-dessus du tube de fourreau (3).

10. Procédé continu de fabrication de produits oxo C₇-C₂₁ par hydroformylation d'au moins une oléfine C₆-C₂₀ avec du gaz de synthèse en présence d'un catalyseur de métal de transition dissous de façon homogène, **caractérisé en ce que** l'on exécute le procédé dans un réacteur cylindrique (1) avec un axe longitudinal vertical,
- avec une multiplicité de tubes Field (2), qui sont orientés parallèlement à l'axe longitudinal du réacteur (1) et qui sont soudés dans une plaque à tubes à l'extrémité supérieure du réacteur (1),
- avec un tube de fourreau (3) ouvert aux deux extrémités, qui entoure les tubes Field (2) et qui dépasse ceux-ci à son extrémité inférieure,
- avec une buse d'admission (4) ou plusieurs buses d'admission (4) au fond du réacteur (1) pour injecter un mélange d'éduit, qui contient l'oléfine C₆-C₂₀, le gaz de synthèse et éventuellement le catalyseur de transition, dans lequel
- les tubes Field (2) sont conçus, en nombre et en dimensions, de telle manière que
- la surface totale de transfert de chaleur de ceux-ci rapportée au volume intérieur du réacteur se situe dans la plage de 1 m²/m³ à 12 m²/m³, et
- la surface de section transversale occupée par les tubes Field (2) rapportée à la surface de section transversale du tube de fourreau (3) se situe dans la plage de 0,03 m²/m² à 0,30 m²/m².

11. Procédé selon la revendication 10, **caractérisé en ce que** le catalyseur de métal de transition est un catalyseur complexe de carbonyle de métal, de préférence un catalyseur complexe de carbonyle de cobalt.
